# EUROPEAN PATENT SPECIFICATION

(11) **EP 1 628 659 B1**
(45) Date of publication and mention of the grant of the patent: **31.12.2008**
(21) Application number: 04732600.4
(22) Date of filing: 13.05.2004
(51) Int. Cl.: A61K 31/4025, A61K 47/40, A61P 33/00

(54) **USE OF POLY-AMINOPYRROLECARBOXAMIDES IN COMBINATION WITH A CYCLODEXTRIN IN THE PROPHYLAXIS AND TREATMENT OF ANIMAL PARASITOSIS**
VERWENDUNG VON POLY-AMINOPYRROLCARBOXAMIDE IN KOMBINATION MIT EINEM CYCLODEXTRIN ZUR PROPHYLAXE UND BEHANDLUNG VON PARASITOSE BEI TIEREN
UTILISATION DE POLY-AMINOPYRROLECARBOXAMIDES EN COMBINAISON AVEC UNE CYCLODEXTRINE POUR PREVENIR ET TRAITER LA PARASITOSE DES ANIMAUX

(30) Priority: 20.05.2003 IT MI20031015
(43) Date of publication of application: 01.03.2006
(73) Proprietor: Naxospharma S.R.L., 20020 Cesate (IT)
(72) Inventor: LOMBARDI, Paolo, I-20020 CESATE (IT)
(74) Representative: Modiano, Micaela Nadia
(86) International application number: PCT/EP2004/005157
(87) International publication number: WO 2004/103363

(56) References cited:
- WO-A-20/04004691
- US-A- 5 472 976
- US-A- 5 670 534
- US-A- 5 712 260

## Description

### Field of the invention

The present invention relates to the use of a compound having the following formula (I): 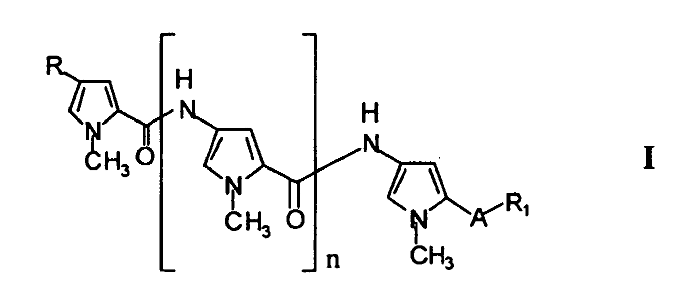 wherein:
n is 0 or an integer comprised between 1 and 5;
R is a group R₂-X -C(=Z)-NH-, in which X represents a simple chemical bond, an aromatic or heteroaromatic radical, Z represents an oxygen atom or the NH group; and:
   if X is a simple chemical bond, R₂ is an hydrogen atom, an alkyl, dialkylaminoalkyl, alkenyl, cycloalkyl, arylalkyl, arylalkenyl, haloalkyl, or an aromatic or heteroaromatic radical;
   if X is an aromatic or heteroaromatic radical, R₂ is nitro, amino or formylamino;
      or:
R is a group R₃-C(=Z)-, in which Z represents an oxygen atom or the NH group, and R₃ represents a hydrogen atom, the -OR₄ or -NR₅R₆ group, and where:
   R₄ is chosen from the group consisting of a hydrogen atom, an alkyl, cycloalkyl, arylalkyl, or an aromatic radical;
   R₅ and R₆, either the same or different, are chosen from the group consisting of a hydrogen atom, an alkyl, cycloalkyl, arylalkyl, aromatic or heterocyclic radical, optionally substituted with a formylamino or a carbamoyl group; or
   R₅ and R₆, joined together form an alkylene group, or the group -(CH₂)₂-O-(CH₂)₂- or the group -(CH₂)₂-NH-(CH₂)₂-;

A represents a simple chemical bond or the group -CO-NH-Y-, wherein Y is an alkylene or aromatic radical;
R₁ is chosen from the group consisting of CH₂N(CH₃)₂, -COOR₄, -B-NR₅R₆, -C(=NH)-NH₂, a heterocyclic radical containing nitrogen, wherein:
   R₄, R₅ and R₆ are as defined above, B represents a simple chemical bond or the -C=O group, and:
      when R₁ is -B-NR₅R₆, and B is a simple chemical bond, or when R₁ is a heterocyclic radical, A is not a chemical bond;
      or a pharmaceutical acceptable salt thereof in association or combination with a cyclodextrin in the manufacture of a veterinary pharmaceutical composition For the prophylaxis and/or treatment of endoparasitosis in animals.

When: n is 1, and R represents the group R₂-X -C(=Z)-NH-, in which X is a chemical bond, R₂ is hydrogen and Z is oxygen, and A represents the group -CO-NH-Y, in which Y is -CH₂-CH₂-, and R₁ represents the group -C(=NH)-NH₂, the compound of formula (I) is the well known antiviral antibiotic Distamycin, isolated from and produced by the mycelium of *Streptomyces distallicus* [A.M. Casazza et al., Antimicrobial Agents Chemother, 5:593 (1965)]. Compounds of general formula (I) are known compounds the preparation of which has been disclosed [ F. Arcamone, et al., Gazz. Chim. Ital. 97:1110 (1967); G. DiPietro et al., Perkin Comm., 1996:1333; P. Lombardi et al., Pharmacol. Ther., 76:125 (1997); F. Animati et al., J. Med. Chem., 38;1140 (1995); WO 9209574; US 5472976; US 5670534]. For the compounds of formula (I) there are generically mentioned various pharmacological activities, e. g. antiviral and antimalarial activities, but said compounds have not found use in the clinical practice. In the previous art, however, there are no teachings of a specific activity of the compounds of the invention in the treatment of endoparasitosis in animals or of their use in veterinary medicine.

### Background of the invention

It would be highly desiderable to accomplish further progress in order to achieve more efficient treatments, free from undue side effects, against the numerous animal parasitoses. At present, there are as yet no reliable therapies for many of those, due to lack of specific drugs; development of resistance to current drugs and of immunodepression in the hosts; widespread resistance of parasites to different environmental conditions, including chemicals usually employed as disinfectants.

Parasites are pathogenic agents by definition, perpetually causing to the host damages which may become more or less severe in relation to the pathogenicity of parasites, their prevalence and the susceptibility of the host, resulting in the onset of diseases which are very often chronic. Moreover, parasites favour the occurrence of other pathogenic agents resulting in more severe conditions, since modifications caused by the parasite may assist infections by other pathogenic microrganisms.

In the case of production or farm animals, this results in reduced yield and quality of products (such as milk, meat, wool and their derivatives) causing significant economic losses to the rearing and production markets, and potential health implications for consumers.

Accordingly, in recent years parasitic infections are major problems in zootechnics, since parasitic infections have become emerging pathologies as a consequence of new production improvement techniques, modem farm management, and breeds of animal with better track records in productivity. In these conditions, the opportunistic nature typical of parasites finds the most favourable conditions to expand its pathogenic potential since the rate of subject-to-subject transmission (particularly high in the confinement rearing which determines a large number of animals permanently housed in restricted spaces) and the decrease of the specific immune responses of the host (bound to a series of factors which characterize intensive farming) allow for the rapid replication and spread of the parasite, resulting in very high prevalences of infection in affected farms, turning into a perpetual threat to animal health and productivity.

In this context, endoparasitoses and, particularly, parasitoses associated with unicellular enteropathogenic parasites, are object of major interest in the medical veterinary practice and research, both for infectivity, morbidity and mortality characteristics in different hosts and for the wide diffusion and capacity to sustain severe zoonosic infections which may result in the death of immunocompromised subjects. Moreover, for many causative agents, the lack of host-specificity determines numerous, actually uncontrollable routes of transmission with respect both to animals and humans. The same parasitic species can be detected in tens of farm, companion or wild animal species, and world-wide.

Important or emerging parasitic pathologies are, for example:
Trichomoniasis, which afflicts birds (turkeys, chickens, etc.) caused by *T. gallinae* and *T. gallinarum*, and infections by *Hexamita meleagridis*, which colonize the upper part of the digestive tract and the distal part of the intestine;
Giardiasis, found world-wide and potentially present in all animals, caused by parasites of which the most common are *G. lamblia* or *G. enterica, G. canis, G. cati, G. bovis, G. duodenalis, G. chinchillae* (particularly severe in chinchilla farms), *G. intestinalis*, localized in the duodenum and colon;
Istomoniasis, or infectious enteropathitis, caused by *Histomonas meleagridis*, localized in the coecum of birds, particularly of turkeys;
Amoebiasis, caused by parasites of the genus Entamoeba, such as for instance *E. histolytica*, localized in the large intestine (coecum and colon), which particularly afflicts humans, but which may be frequently present in animals, which are reservoirs of infection for humans;
Coccidiosis, which can develop in almost all domestic and wild animals, and even in humans, found world-wide and also endemically, caused by families of numerous intestinal parasites, such as species belonging to the genus Eimeria, for example, *E. bovis, E. ellipsoidalis*, *E. necatrix, E. tenella, E. stiedae, E. alabamensis, E. perforans*, and *E. zuernii*; species belonging to the genus Isospora, for example, *I*. *hominis, I. belli, I. bigemina*, *I. rivolta, I*. *felis*; species belonging to the genus Cryptosporidium, for example, *C. parvum, C. muris, C.bailey, C. felis, C. canis, C. meleagridis, C. nasorum, C. serpentis, C. wrairi, C. andersoni, C. blagburni, C. saurophilum*, *C. molnari*;
Balantidiosis, caused by *Balantidium coli*, localized in the large intestine of pigs, but also of cattle and humans.

As a consequence of present investigation techniques, new aspects concerning the genetic polymorphism of the above mentioned parasites are continuosly elucidated and new classifications are set forth, therefore the above mentioned parasitic species are only given for purpose of exemplification and their number is subjected to increase.

Although many chemotherapeutic compounds have been tested for efficacy against enteropathogenic parasites in animals, there is no clearly recognized, widely acccepted, or immediately available compounds for prophylaxis or therapy. Some drugs have demonstrated to improve in part the symptomatology and rate of mortality within a farm, others turned to be toxic to the animal at the therapeutically effective dose.

USP 5 670 534 discloses distamycin compounds and methods of use and their pharmaceutical compositions.

USP 5 712 260 discloses a pharmaceutical composition comprising an estramustine derivative and a cyclodextrin, particularly in the manufacture of a medicament suitable for the oral administration of an estramustine derivative to a patient suffering from a tumor.

WO 2004/004691 Al discloses a pharmaceutical formulation constituted by a lexitropsin phospholipidic complex in the form of liposomes, micelles or nanoparticles that exhibit optimal pharmacological properties in both the tropical or parenteral treatment of infectious and/or tumor diseases.

### Detailed description of the invention

It has been found that the compounds of formula (I) according to the present invention, besides exhibiting antiviral and antimalarial activities, surprisingly exhibit at various degrees, and according to the different substituents, in vitro and in vivo antiparasitic activities with respect to internal parasites which afflict animals, in particular with respect to parasites located in the gastro-intestinal tract (enteropathogenic or intestinal parasites), which are the causative agents of several, and sometimes severe, enteric infections and enteritis, as mentioned herebefore.

The water solubility of the pharmaceutically acceptable salts of the compounds of formula (I) and the surprising absence of oral bioavailability, which renders the inventive compounds substantially safe and non-toxic with respect to the recipient animal, allow the compounds of the invention to achieve the required therapeutic and prophylactic action against the above-mentioned parasites in the cases both of acute infection and when there is a need to keep the parasite population under a safe control.

It is known, in fact, that affected animals often reject food, but very rarely refuse to drink. It is also known that several drugs used in the veterinary practice, and particularly drugs used to control parasites, may be found as such, or as metabolites thereof, in the animal products for human use (meat, milk).

Therefore, the compounds of the present invention exhibit a remarkable useful advantage with respect to many drugs of the prior art in that they can be administered as dissolved in drinking water and, not been adsorbed across the gastrointestinal tract when administered to the patient orally, they neither diffuse in the animal tissues nor are here metabolized.

The pharmaceutical acceptable salts of the compounds of formula (I) are the salts by addition of pharmaceutically acceptable inorganic acids, such as for instance hydrochloric, sulphuric, and phosphoric acid, as well of pharmaceutically acceptable organic acids, such as for instance acetic, maleic, ascorbic, succinic, benzoic, and salicilic acid.

Particularly preferred are the salts by addition of hydrochloric acid.

Preferred examples of the compounds according to the present invention are:
distamycin; and the compounds of formula (I) wherein:
   n is as previously defined;
   R is the -CONH₂ group, A is the -CONHCH₂CH₂- group, R₁ is the -C(=NH)-
   NH₂ group or the -CH₂N(CH₃)₂ group;
   R is the -NH-CH(=NH) group, A is the -CONHCH₂CH₂- group, R₁ is the C(=NH)-NH₂ group or the -CH₂N(CH₃)₂ group or the -CONH₂ group;
   and the pharmaceutically acceptable salts thereof.

The compounds of formula (I) appear not to be orally bioavailable, as set forth in the Example 3, where for the only purpose of exemplification, there is presented a study comparing blood concentrations of a compound of the invention in the rat following intravenous (i. v.) and oral (p. o.) administration, respectively.

The pharmaceutical composition of the present invention, therefore, finds useful application in the prophylaxis and therapeutic treatment of endoparasitosis in animals, in particular production and farm animals, including mammals, such as cattle, sheep, goats, pigs and horses, and non-mammals, such as chickens, turkeys, pidgeons, ducks, geese, quails, phesants, as well as companion pets, such as dogs, cats, and cage birds.

Preferred examples of parasite-caused enteric diseases which can be prevented or treated with the pharmaceutical composition of the invention are Trichomoniasis, Giardiasis, Istomoniasis, Amoebiasis, Coccidiosis, and Balantidiosis.

The pharmaceutical composition of the present invention is preferably administered orally to the animal, for instance as dissolved in drinking water or admixed with food.

The compounds according to the invention can be administered to animals orally in various solid or non-solid forms, such as, for instance, tablets, capsules, tablets coated with films or sugar, solutions or suspensions, preferably as solutions, preferably dissolved in water.

The dosage is dependent on the age, weight, general condition, species of the animal and on the route of administration, for instance by bolus, dispersion in the drinking water or in the food. For example, an effective dosage for an adult animal can be comprised between 0.1 and 500 mg/kg pro dose administered from 1 to 5 times per day, or can be a concentration comprised between 0,5 and 5 g/L drinking water or can be an amount comprised between 1 and 10 g/kg food, according to the judgement of the attending veterinary physician.

Pharmaceutical compositions comprising the compounds of the invention are prepared according to conventional techniques. Solid dosage forms may contain, for instance, in addition to the active principle, diluents such as lactose, dextrose, sucrose, cellulose, potato or cereal starches; lubricants such as silicium oxide, talc, stearic acid, calcium stearate, magnesium stearate and/or polyethylene glycols; binders such as starches, arabic gum, gelatin, methylcellulose, carboxymethylcellulose or polyvinylpyrrolidinone; disintegrating agents such as starch, alginic acid, alginates and amides, sodium glycolate; dyes; sweeteners; wetting agents such as lecithin, polysorbates, laurylsulphates; and generally non-toxic and pharmaceutically inactive substances such as those conventionally used in pharmaceutical formulations. Such pharmaceutical preparations can be made up according to known methods, e. g. by mixing, grinding, tabletting, coating processes. Liquid dispersions may be for instance syrups, solutions, emulsions and suspensions. Syrups and solutions may contain carriers such as sucrose, glycerol, mannitol, sorbitol. Suspensions and emulsions may contain carriers such as a gum, agar, sodium alginate, pectin, methylcellulose, carboxymethylcellulose, polyvinyl alcohol.

The object of the present invention is a pharmaceutical composition comprising a compound according to the invention in association and/or combination with a cyclodextrin. Cyclodextrins (hereinafter CD) are well known cyclic oligosaccharides, made up of D-glucose residues, having a cylindrical cavity shaped structure capable of including various molecules (guest molecules). One of the most interesting properties of CD is their ability to form inclusion compounds or complexes. This kind of complexation confers new physicochemical properties to guest molecules, and is extensively used and exploited in the pharmaceutical field to improve the solubility and stability of pharmacologically active substances (O. Beckers et al., Drug Dev. Ind. Pharm., 17:1503 (1991); J. Szejtli, Pharm. Tech. Int., February 1991: 15) and, accordingly, their dissolution characteristics and bioavailability.

Said inclusion complexes are usually prepared in a liquid medium, such as water or mixtures of water and organic solvents, and are obtained as powders upon drying.

A variety of methods are known in the art for preparing solid inclusion compounds, such as kneading (K. Uekama et al., Int. J. Pharm., 10:1 (1982)); co-precipitation (K. Uekama et al., Int. J. Pharm., 16:327 (1983)); spray-drying (H.P.R. Bootsma et al., Int. J. Pharm., 51:213 (1989); freeze-drying (US Pat. No. 4,603,123). In some cases the formation of the complex in the solid phase is thermodinamically spontaneous and inclusion could be achieved by simply grinding (C. Torricelli et al., Int. J. Pharm, 71:19 (1991)).

Moreover, it is known that the effectivity of certain drugs can be still improved with CD also for molecules which are highly soluble in water (such as the pharmaceutically acceptable salts of the compounds of the invention), that theoretically do not need any particular formulation intended for improving their solubility or their rate of dissolution (US Pat. No. 5,712,260).

In the instant object, the general terms "cyclodextrin" and "CD" refers to naturally-occurring (α-CD, β-CD and γ-CD), synthetic or semi-synthetic (e. g. hydroxypropyl-β-CD or dimethyl-β-CD) cyclodextrins. Particularly preferred CDs are β-cyclodextrin, hydroxypropyl-β-cyclodextrin, and γ-cyclodextrin.

It has been surprisingly found that when the pharmaceutically acceptable salts of the compounds of formula (I) are mixed with a cyclodextrin, the solubility of the compounds according to the invention does not change significantly, but the effects of reprecipitation induced by ionic species or pH changes on the compounds of formula (I) are negligible, since cyclodextrins are able to mask the site of interaction of the compounds of formula (I).

Thus, the formation of a complex in solution between the compounds of the invention and an appropriate cyclodextrin may avoid the incidental reprecipitation of the compounds of the invention either as free bases or as insoluble salts in physiological conditions. This phenomenon can result in a big advantage in the administration of the compounds according to invention by oral route, because their antiparasitic efficacy is not affected by the environment of the gastroenteric tract (stomach, bowel) and by their particular conditions (pH, presence of ionic species which may render the compounds of the invention insoluble, fasting/non-fasting conditions of the subject animal during treatment).

Moreover, it has been noticed that it is not necessary to form the complex between a compound of formula (I) and a CD in the solid state, but it is sufficient to administer a simple physical mixture of the two chemical entities, i. e., a combination or association thereof.

The proportion between a compound of formula (I) and a CD may vary, e. g., from 1:0,5 to 1:10 as molar ratio, preferably from 1:1 to 1:4.

The following Examples are only intended to illustrate the invention without limiting the same.

### Example 1

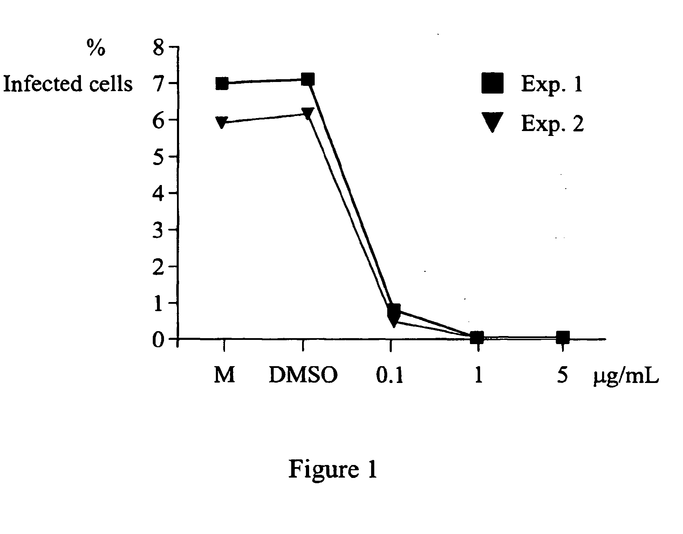

In vitro activity. CACO2 cell cultures (2 x 10⁶ cells 70% confluent) were inoculated with purified sporozooites of *C. parvum* and, at the time of infection, treated with either variable concentrations of hydrochloride of the compound of formula (I, R = -CONH₂, A = -CONHCH₂CH₂-, R₁ = -C(=NH)-NH₂) dissolved in dimethylsulphoxide (DMSO) or DMSO (5% medium) in controls. The test compound was removed 30 minutes later by repeated washings. The percentage of infected cells was determined 24 hours after beginning of experiment by microscopic examination after Giemsa staining, analysing 10 microscopic fields for each experimental point at 400x. The values obtained for each point represent the mean of three independent replicates. The percentage of infected cells in untreated controls (i. e., treated with DMSO only) was 7.1% for Experiment 1, and 6.35 for experiment 2. Results set forth in Figure 1 show a remarkable cytotoxic activity of the test compound against the object parasite.

### Example 2

In vivo activity. The in vivo oral antiparasitic activity of the compound of formula (I, R = -CONH₂, A = -CONHCH₂CH₂-, R₁ = -C(=NH)-NH₂) was assessed by evaluating the ability of test compound to modify the course of experimentally induced infection in immuno-suppressed BALB/C mice. After several weeks of immunosuppressive treatment with dexamethasone, 30 mice were orally infected with *C. parvum* oocysts on the same day. Two weeks after infection, mice shedding oocysts, identified by detecting oocysts in the stools using both Zeil Niessel staining and immunofluorescence, were pooled in three groups of 5 animals each. Two groups were treated with hydrochloride of the test compound dissolved in drinking water at a concentration of 5 µg/mL (mice are expected to drink about 2 -5 mL of water per day), whereas the third group (control group) did not receive any treatment. To evaluate the efficacy of the treatment, the shedding of oocysts was monitored for 4 weeks. Animals receiving treatment showed a significant reduction in oocyst shedding after 1 week, and oocysts could not be detected after 2 weeks of treatment with the test compound, and remained parasite free until the end of the experiment (4 weeks).

### Example 3

10 male Sprague-Dawley rats weighing 320 - 390 g were used with the scope to determine plasma levels of the compound of formula (I, R = -CONH₂, A = - CONHCH₂CH₂-, R₁ = -C(=NH)-NH₂) after intravenous and oral administration, respectively. Rats were anesthetized and the right jugular vein was cannulated and the cannula was left exposed on the neck to allow for drug administration and blood collection. Rats were administered 24 - 48 hours after recovery from surgical anesthesia.

Intravenous drug administration (10 mg/kg). 10 mg of hydrochloride of the test compound were dissolved in a vehicle constituted of DMSO and saline buffer (1 : 20 v/v) at a concentration of 1.5 mg/mL. The administered volume was 2 mL/kg as bolus.

Oral administration (20 mg/kg). 20 mg of hydrochloride of the test compound were dissolved in a vehicle constituted of DMSO and saline buffer (1 : 250 v/.v) at a concentration of 5 mg/mL. The administered volume was 5 mL/kg.

Sample collection. Blood samples (400-500 µl) were withdrawn from jugular vein at intervals of 0, 5, 15, 30, 60, 90, 120, 180, 240, 360, 480, 680 and 1440 minutes after intravenous and oral administration, respectively, of test drug. The amount of collected blood was replaced by an equal volume of saline.

Plasma levels. The determination of test compound blood levels was performed by using a validated HPLC method. The limit of quantitation of the assay was 0.1 µg/0.5 mL.

After a single intravenous administration of 10 mg/kg of the drug, the mean drug concentration in blood declined according to a biexponential profile.

After a single oral administration of 20 mg/kg of the drug, no detectable amount of the drug was found in blood in the time interval 0 - 1440 minutes, indicating that the test compound is not orally adsorbed. Data are set forth in Table 1.

**Table 1**

| min | i.v. (µg/ml) | p.o. |
|---|---|---|
| 0 | Nd | nd |
| 5 | 10.0 ± 0.95 | nd |
| 15 | 8.2 ± 0.98 | nd |
| 30 | 6.9 ± 0.67 | nd |
| 60 | 5.2 ± 0.65 | nd |
| 90 | 3.8 ± 0.59 | nd |
| 120 | 2.7 ± 0.74 | nd |
| 180 | 1.5 ± 0.44 | nd |
| 240 | 1.1 ± 0.21 | nd |
| 360 | 0.89 ± 0.15 | nd |
| 480 | 0.72 ± 0.09 | nd |
| 680 | 0.53 ± 0.16 | nd |
| 1440 | 0.27 ± 0.11 | nd |

### Example 4

0.15 mmol of hydrochloride of a compound of formula (1) and 0.38 mmol of β-cyclodextrin are dissolved in 100 mL of water with stirring for 6 hours. The resulting product is separated by precipitation by cooling the solution at 3°C or, alternatively, by freeze-drying the solution.

### Example 5

2,64 mmol of β-cyclodextrin are dissolved in 100 mL of water by applying a gentle heating. To the resulting solution there are added 1,06 mmol of hydrochloride of a compound of formula (I) with stirring for 6 - 12 hours. The product is separated by precipitation by cooling the solution at 3°C or, alternatively, by freeze-drying the solution.

### Example 6

Mixtures of hydrochloride of a compound of formula (I) and of different amounts of β-cyclodextrin, or 2-hydroxypropyl-β-cyclodextrin, or γ-cyclodextrin are prepared by grinding, followed by sieving (<0.375 mm) and 10 minute of mixing in a turbo-mixer.

## Claims

1. Use of a compound having the following formula (I): 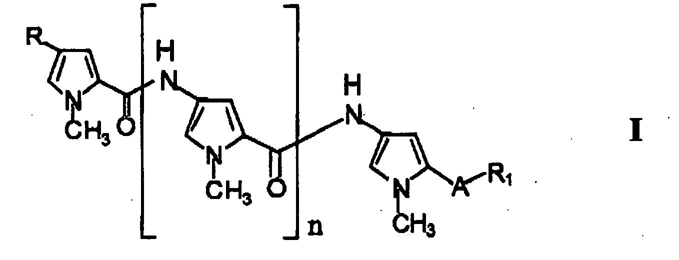 wherein:
n is 0 or an integer comprised between 1 and 5;
R is a group R₂-X -C(=Z)-NH-, in which X represents a simple chemical bond, an aromatic or heteroaromatic radical, Z represents an oxygen atom or the NH group; and:
if X is a simple chemical bond, R₂ is an hydrogen atom, an alkyl, dialkylaminoalkyl, alkenyl, cycloalkyl, arylalkyl, arylalkenyl, haloalkyl, or an aromatic or heteroaromatic radical;
if X is an aromatic or heteroaromatic radical, R₂ is nitro, amino or formylamino;
or:
R is a group R₃-C(=Z)-, in which Z represents an oxygen atom or the NH group, and R₃ represents a hydrogen atom, the -OR₄ or -NR₅R₆ group, and where:
R₄ is chosen from the group consisting of a hydrogen atom, an alkyl, cycloalkyl, arylalkyl, or an aromatic radical;
R5 and R6, either the same or different, are chosen from the group consisting of a hydrogen atom, an alkyl, cycloalkyl, arylalkyl, aromatic or heterocyclic radical, optionally substituted with a formylamino or a carbamoyl group; or
R₅ and R6, joined together form an alkylene group, or the group -(CH₂)₂-O-(CH₂)₂- or the group -(CH₂)₂-NH-(CH₂)₂-;
A represents a simple chemical bond or the group -CO-NH-Y-, wherein Y is an alkylene or aromatic radical;
R₁ is chosen from the group consisting of CH₂N(CH₃)₂, - COOR₄, -B-NR₅R₆, - C(=NH)-NH₂, a heterocyclic radical containing nitrogen, wherein:
R₄, R₅ and R₆ are as defined above, B represents a simple chemical bond or the -C=O group, and:
when R₁ is -B-NR₅R₆, and B is a simple chemical bond, or when R₁ is a heterocyclic radical, A is not a chemical bond;
or a pharmaceutical acceptable salt thereof in association or combination with a cyclodextrin in the manufacture of a veterinary pharmaceutical composition for the prophylaxis and/or treatment of endoparasitosis in animals.

2. Use according to Claim 1, where the compound of formula (I) is chosen between distamycin and a compound of formula (I) wherein:
n is as previously defined;
R is the -CONH₂ group, A is the -CONHCH₂CH₂- group, R₁ is the -C(=NH)-NH₂ group or the -CH₂N(CH₃)₂ group;
R is the -NH-CH(=NH) group, A is the -CONHCH₂CH₂- group, R₁ is the -C(=NH)-NH2 group or the -CH₂N(CH₃)₂ group or the -CONH₂ group;
and the pharmaceutically acceptable salts thereof.

3. Use according to Claim 1, where the endoparasitosis is chosen from Trichomoniasis, Giardiasis, Istomoniasis, Amoebiasis, Coccidiosis, and Balantidiosis.

4. Use according to Claim 1, where the pharmaceutical composition is for oral use.

## Patentansprüche

1. Verwendung einer Verbindung mit der folgenden Formel (I): 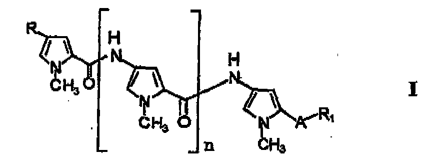 worin:
n 0 ist oder eine ganze Zahl zwischen 1 und 5;
R ist eine Gruppe R₂-X-C(=Z)-NH, worin X eine einfache chemische Bindung darstellt, ein aromatisches oder heteroaromatisches Radikal, Z stellt ein Sauerstoffatom oder die NH-Gruppe dar; und:
wenn X eine einfache chemische Bindung ist, ist R₂ ein Wasserstoffatom, ein Alkyl, Dialkylaminoalkyl, Alkenyl, Cycloalkyl, Arylalkyl, Arylalkenyl, Haloalkyl oder ein aromatisches oder heteroaromatisches Radikal;
wenn X ein aromatisches oder heteroaromatisches Radikal ist, ist R₂ Nitro, Amino oder Formylamino;
oder:
R ist ein Gruppe R₃-C(=Z)-, worin Z ein Sauerstoffatom oder die NH-Gruppe darstellt, und R₃ stellt ein Wasserstoffatom, die -OR₄ oder -NR₅R₆-Gruppe dar, und worin:
R₄ gewählt ist aus der Gruppe bestehend aus einem Wasserstoffatom, einem Alkyl, Cycloalkyl, Arylaklyl, oder einem aromatischen Radikal;
R₅ und R₆, entweder gleich oder unterschiedlich, sind gewählt aus der Gruppe bestehend aus einem Wasserstoffatom, einem Alkyl, Cycloalkyl, Arylalkyl, aromatischen oder heterozyklischen Radikal, wahlweise substituiert mit einer Formylamino- oder einer Carbamoylgruppe;
oder
R₅ und R₆ bilden zusammengenommen eine Alkylengruppe, oder die Gruppe -(CH₂)₂-O-(CH₂)₂- oder die Gruppe -(CH₂)₂-NH-(CH₂)₂-; A stellt eine einfache chemische Bindung dar, oder die Gruppe -CO-NH-Y-, worin Y ein Alkylen oder ein aromatisches Radikal ist;
R₁ ist gewählt aus der Gruppe bestehend aus CH₂N(CH₃)₂, -COOR₄, -B-NR₅R₆, -C(=NH)-NH₂, einem heterozyklischen Radikal enthaltend Stickstoff, worin:
R₄, R₅ und R₆ wie oben definiert sind, B stellt eine einfache chemische Bindung dar oder die -C=O Gruppe, und:
wenn R₁ -B-NR₅R₆ ist, und B ist eine einfache chemische Bindung, oder wenn R₁ ein heterozyklisches Radikal ist, ist A keine chemische Bindung;
oder ein pharmazeutisch verträgliches Salz davon in Verbindung oder Kombination mit einem Cyclodextrin in der Herstellung einer tierärztlichen pharmazeutischen Zusammensetzung für die Prophylaxe und / oder die Behandlung von Endoparasitose in Tieren.

2. Verwendung gemäß Anspruch 1, worin die Verbindung der Formel (I) gewählt ist zwischen Distamycin und einer Verbindung der Formel (I), worin:
n wie zuvor definiert ist;
R die -CONH₂-Gruppe ist, A die -CONHCH₂CH₂-Gruppe ist, R₁ die -C(=NH)-NH₂-Gruppe oder die -CH₂N(CH₃)₂-Gruppe ist;
R ist die -NH-CH(=NH)-Gruppe, A ist die -CONHCH₂CH₂-Gruppe, R₁ ist die -C(=NH)-NH₂-Gruppe oder die -CH₂N(CH₃)₂-Gruppe oder die -CONH₂-Gruppe;
und die pharmazeutisch verträglichen Salze davon.

3. Verwendung gemäß Anspruch 1, worin die Endoparasitose gewählt ist aus Trichomoniasis, Giardiasis, Istomoniasis, Amoebiasis, Coccidiosis und Balantidiosis.

4. Verwendung gemäß Anspruch 1, worin die pharmazeutische Zusammensetzung für die orale Verwendung ist.

## Revendications

1. Utilisation d'un composé ayant la formule (I) suivante : 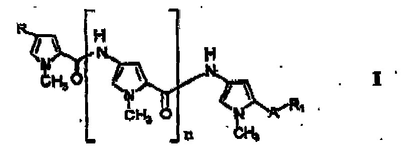 dans laquelle :
n est égal à 0 ou à un nombre entier compris entre 1 et 5 ;
R est un groupe R₂-X-C(=Z)-NH- dans lequel X représente une liaison chimique simple, un radical aromatique ou hétéro-aromatique, Z représente un atome d'oxygène ou le groupe NH ; et
si X est une liaison chimique simple, R₂ est un atome d'hydrogène, un alkyle, un dialkyalminoalkyle, un alcényle, un cycloalkyle, un arylalkyle, un arylalcényle, un haloalkyle, ou un radical aromatique
ou hétéroaromatique ;
si X est un radical aromatique ou hétéroaromatique, R₂ est un nitro, amino ou formylamino ;
ou
R est un groupe R₃-C(=Z)-, dans lequel Z représente un atome d'oxygène ou le groupe NH, et R₃ représente un atome d'hydrogène, le groupe -OR₄ ou - NR₅R₆ et où :
R₄ est choisi dans le groupe constitué d'un atome d'hydrogène, d'un alkyle, d'un cycloalkyle, d'un arylalkyle, ou d'un radical aromatique ;
R₅ et R₆, identiques ou différents, sont choisis dans le groupe constitué d'un atome d'hydrogène, un alkyle, cycloalkyle, arylalkyle, radical aromatique ou hétérocyclique, éventuellement substitué par un groupe formylamino ou carbamoyle ;
ou
R₅ et R₆, joints ensemble, forment un groupe alcylène, ou le groupe -(CH₂)₂-O-(CH₂)₂- ou le groupe - (CH₂)₂-NH-(CH₂)₂- ;
A représente une liaison chimique simple ou le groupe -CO-NH-Y-, dans laquelle Y est un alcylène ou un radical aromatique :
R₁ est choisi dans le groupe constitué de CH₂N(CH₃)₂, -COOR₄, -B-NR₅R₆, -C(=NH)-NH₂, un radical hétérocyclique contenant de l'azote, dans lequel :
R₄, R₅ et R₆ sont comme définis ci-dessus, B représente une liaison chimique simple ou
le groupe -C=O, et
quand R₁ est -B-NR₅R₆ et B est une liaison chimique simple, ou quand R₁ est un radical hétérocyclique, A n'est pas une liaison chimique ;
ou son sel pharmaceutiquement acceptable, en association ou en combinaison avec une cyclodextrine, dans la fabrication d'une composition pharmaceutique vétérinaire pour la prophylaxie et/ou le traitement de l'endoparasitose chez les animaux.

2. Utilisation selon la revendication 1, dans laquelle le composé de formule (I) est choisi parmi la distamycine et un composé de formule (I), dans lequel :
n est comme précédemment défini ;
R est le groupe -CONH₂, A est le groupe - CONHCH₂CH₂-, R₁ est le groupe -C(=NH)-NH₂ ou le groupe - CH₂N(CH₃)₂ ;
R est le groupe -NH-CH(=NH), A est le groupe - CONHCH₂CH₂-, R₁ est le groupe -C(=NH)-NH₂ ou le groupe - CH₂N(CH₃)₂ ou le groupe -CONH₂ ;
et ses sels pharmaceutiquement acceptables.

3. Utilisation selon la revendication 1, dans laquelle l'endoparasitose est choisie parmi la Trichomoniasis, la Giardiasis, l'Istomoniasis, l'Amoebiasis, la Coccidiosis, et la Balantidiosis.

4. Utilisation selon la revendication 1, dans laquelle la composition pharmaceutique est destinée à un usage oral.
